# EUROPÄISCHE PATENTSCHRIFT

(11) **EP 0 718 296 B1**
(45) Veröffentlichungstag und Bekanntmachung des Hinweises auf die Patenterteilung: **19.05.1999**
(21) Anmeldenummer: 95119365.5
(22) Anmeldetag: 08.12.1995
(51) Int. Cl.: C07D 417/12, A61K 31/505

(54) **Thermodynamisch stabile Form von N-[4-[2-(2,4-Dimethylphenoxy)-phenyl]-2-thiazolyl]-1,4,5,6-tetrahydro-2-pyrimidinamin (DTTP)**
Thermodynamically stable form of N-[4-[2-(2,4-dimethyl phenoxy)-phenyl]-2-thiazolyl] -1,4,5,6-tetrahydro-2-pyrimidinamine (DTTP)
Forme thermodynamiquement stable de N-[4-[2- 2,4-diméthyl phénoxy)-phényl]-2-thiazolyle] -1,4,5,6-tétrahydro-2-pyrimidinamine (DTTP)

(30) Priorität: 21.12.1994 DE 4445785
(43) Veröffentlichungstag der Anmeldung: 26.06.1996
(73) Patentinhaber: BAYER AG, 51368 Leverkusen (DE)
(72) Erfinder: Grunenberg, Alfons, Dr., D-41539 Dormagen (DE); Petesch, Nicole, Dr., D-42115 Wuppertal (DE)

(56) Entgegenhaltungen:
- EP-A- 0 365 915
- 'Remington's Pharmaceutical Sciences' 1975 , MACK PUBLISHING COMPANY , EASTON, PENNSYLVANIA * Seite 198 - Seite 199 *
- 'Arzneiformenlehre, Ein Lehrbuch für Pharmazeuten' 1985 , WISSENSCHAFTLICHE VERLAGSGESELLSCHAFT MBH , STUTTGART Seiten 207-211 und 530-533

## Beschreibung

Die Erfindung betrifft eine neue, bei Raumtemperatur thermodynamisch stabile polymorphe Form von DTTP, Verfahren zu ihrer Herstellung, diese enthaltende Arzneimittel sowie ihre Verwendung bei der Bekämpfung von Krankheiten. Die Herstellung und Verwendung von DTTP als Antimykotikum ist bereits aus DE-A-3 839 758 A1 und als Schädlingsbekämpfungsmittel aus DE-A-3 836 161 A1 bekannt geworden.

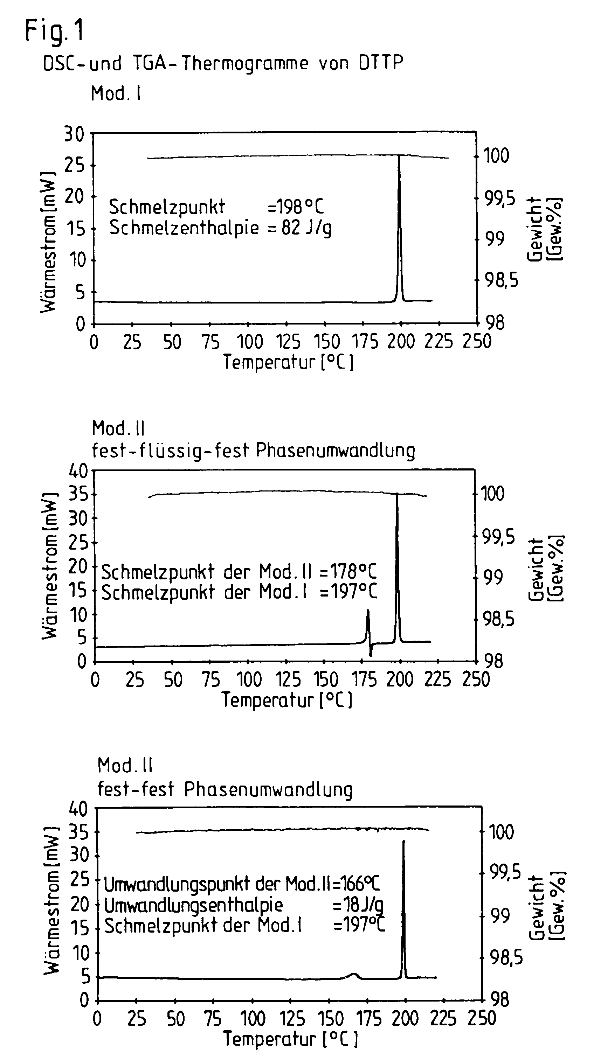

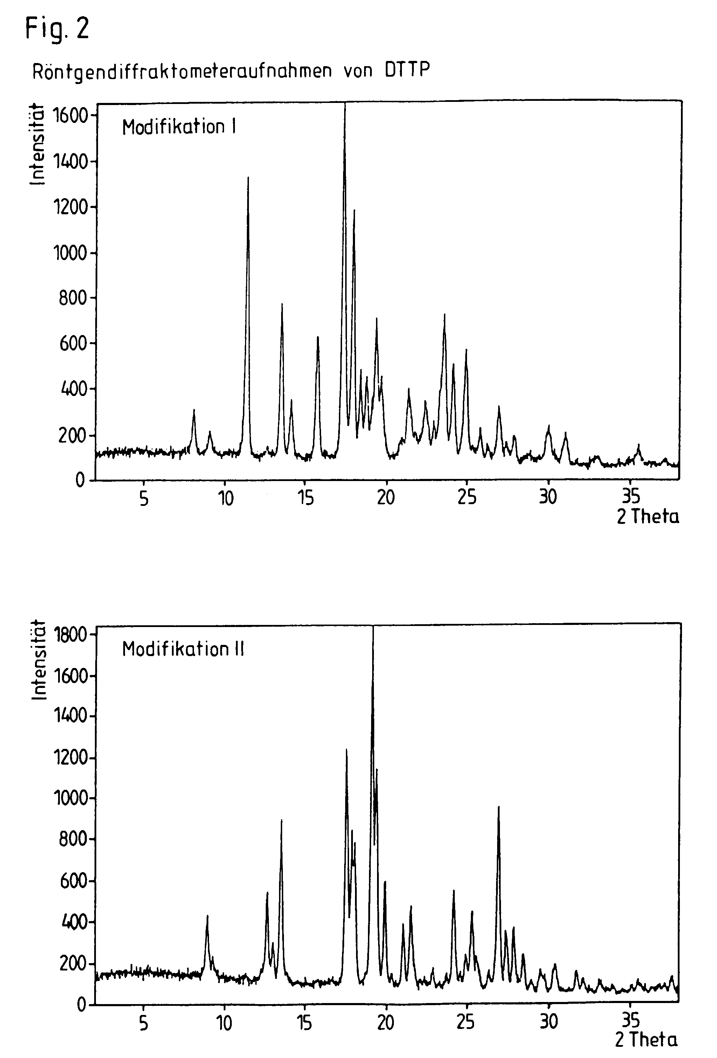

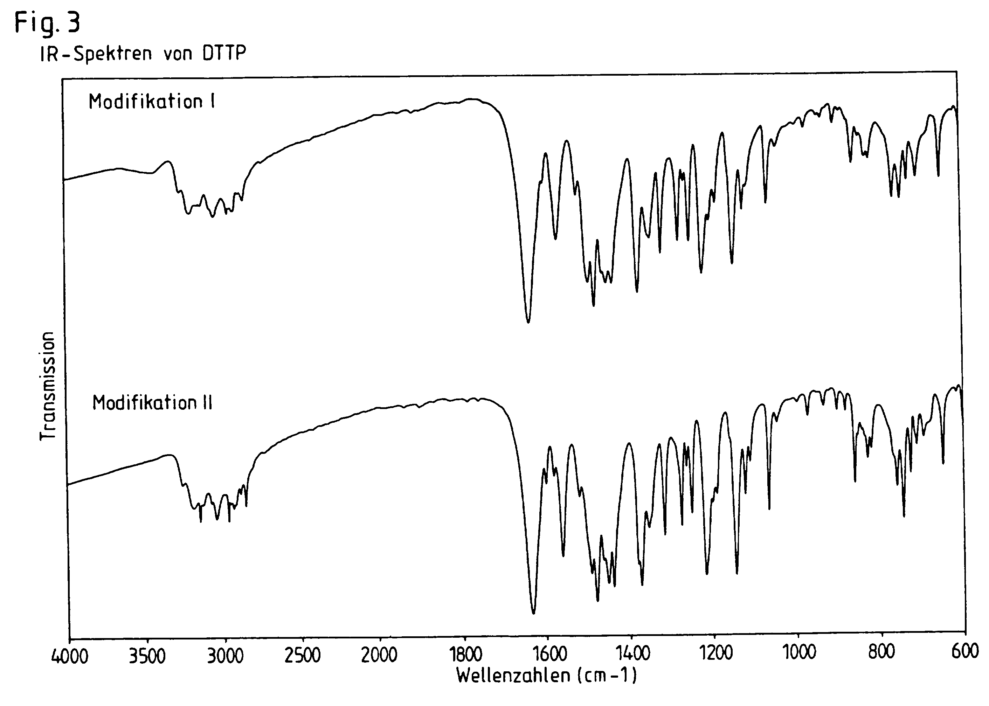

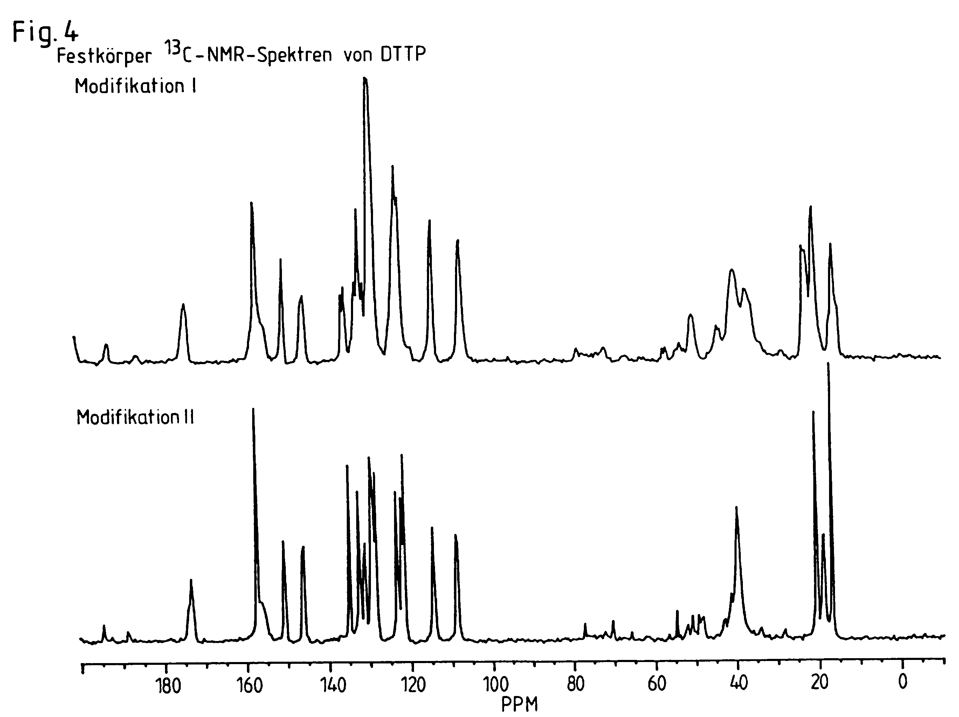

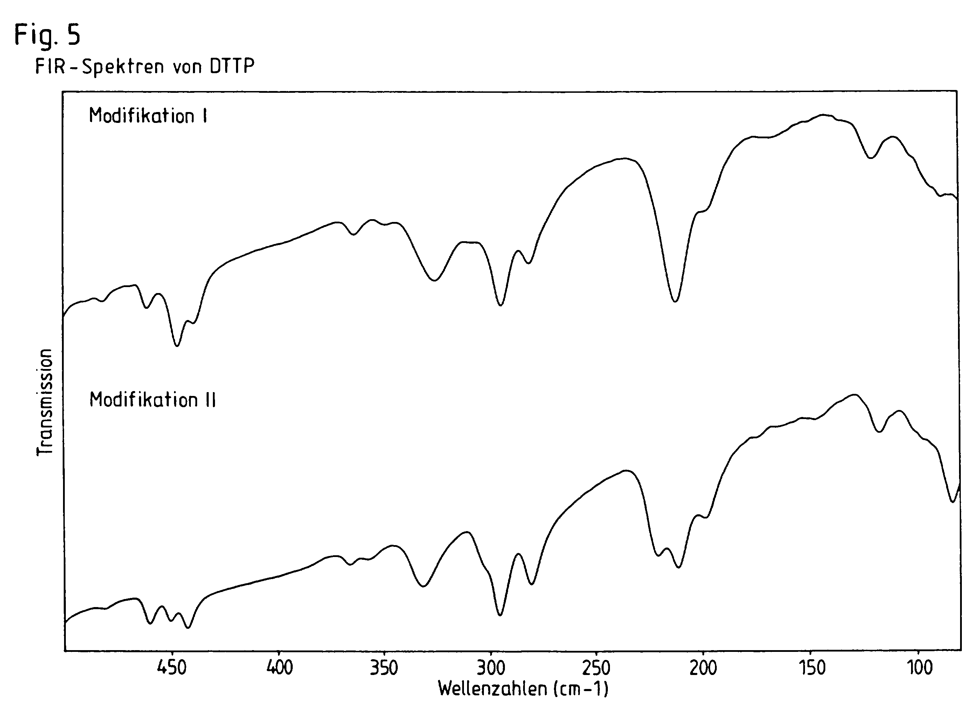

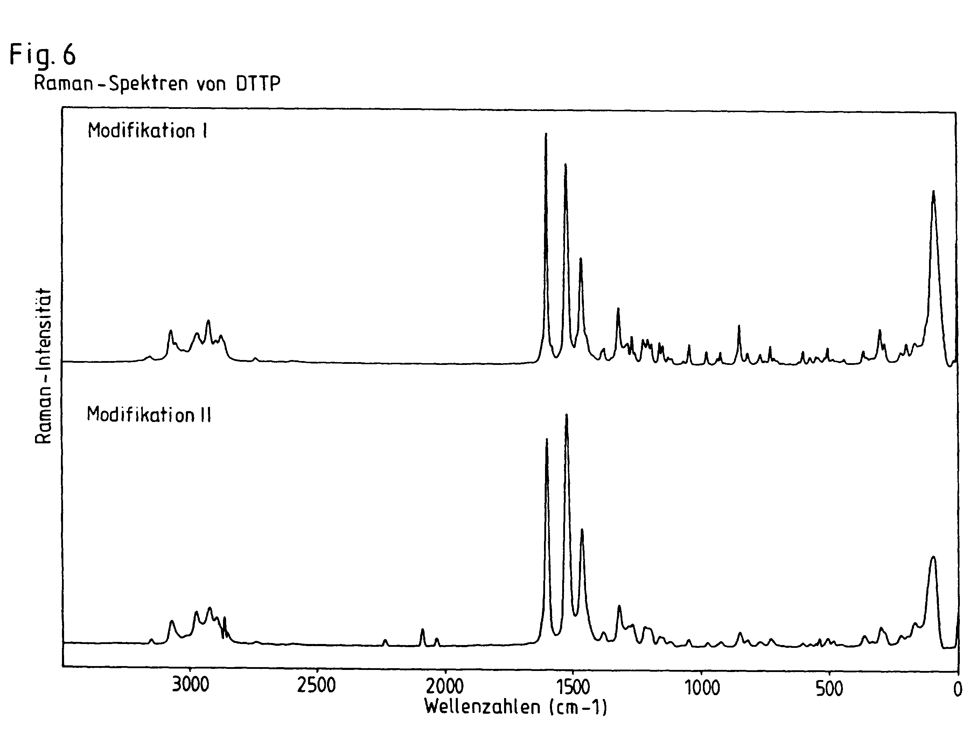

Auf die dort beschriebene Weise wird DTTP in Form einer Kristallmodifikation erhalten, die im folgenden als Modifikation I bezeichnet wird. Durch Umkristallisation gereinigte Substanz der Mod. I hat einen Schmelzpunkt von 198°C (DSC, Heizrate 10 K min⁻¹) und ein charakteristisches Röntgendiffraktogramm, IR-Spektrum, ¹³C-Festkörper-NMR-Spektrum, FIR-Spektrum, Raman-Spektrum (Abb. 1-6). Es wurde nun gefunden, daß Mod. I metastabil ist und deshalb nicht für die Verwendung in pharmazeutischen Formulierungen, wie z. B. Suspensionen und Cremes, geeignet ist.

Überraschenderweise wurde eine zweite Modifikation des DTTP gefunden, die thermodynamisch stabil, weniger hygroskopisch, photochemisch stabiler und auch bei hoher Luftfeuchtigkeit lagerstabil ist und deshalb besonders geeignet ist für den Einsatz in pharmazeutischen Formulierungen, wie z. B. Suspensionen oder Cremes, aber auch zum Einsatz in anderen Zubereitungen, wie z.B. solche zur Verwendung im Materialschutz. Diese neue Modifikation wird im folgenden als Modifikation II bezeichnet. Gegenstand der vorliegenden Erfindung sind auch pharmazeutische Formulierungen, die DTTP in der Modifikation II als aktive Substanz enthalten. Die Formulierung kann eine oder mehrere pharmazeutisch akzeptierte Hilfstoffe, wie. z. B. Binder, Lösungsmittel, Füllstoffe, etc. enthalten.

Aus J. Halbelian, W. McCrowe, J. Pharm. Sci. 58 (1969) 911, ist bekannt, daß beim Einsatz einer thermodynamisch metastabilen polymorphen Form in Suspensionen und Cremes die stabile Form entstehen kann. Als Begleiterscheinung wird dabei unterwünschtes Kristallwachstum, Veränderungen in der Bioverfügbarkeit, Verbackungen usw. beobachtet.

Im Gegensatz zu Modifikation II ist die metastabile Modifikation I hygroskopisch. Bei 85 % rel. Feuchte nimmt Mod. I innerhalb von 2 Stunden 2,8 % Wasser auf. Mod. II sorbiert kein Wasser.

Im Vergleich zu Mod. I ist die Modifikation II des DTTP photochemisch stabiler. Nach 6 Stunden Bestrahlung mit Xenonlicht (globale Bestrahlungsdosis 4810 Wh/m²) wird Gelbfärbung der bestrahlten Oberfläche der Modifikation I beobachtet. Dagegen bleibt unter den gleichen Bestrahlungsbedingungen Modifikation II nahezu weiß.

Mod. II ist im Vergleich zu Mod. I bei hoher Feuchte (85 % r. F., Raumtemperatur) lagerstabil. Mod. I wandelt sich unter diesen Bedingungen nach ca. drei Monaten merklich und nach ca. 6 Monaten zu ca. 40 % in die thermodynamisch stabile Mod. II um.

Durch den erfindungsgemäßen Einsatz der Modifikation II wird die Sicherheit für Zubereitungen des DTTP erhöht und somit das Risiko für den Patienten verringert.

Mod. II hat im Vergleich zu Mod. I ein klar unterscheidbares DSC-Thermogramm, Röntgendiffraktogramm, ¹³C-Festkörper-NMR-Spektrum, FIR-Spektrum und Raman-Spektrum (Abb. 1-6, Tab. 1-6).

Der Schmelzpunkt der Mod. II ist 178°C. Dieser kann jedoch nur bei zügigem Aufheizen einer Probe beobachtet werden (ca. 10 K·min⁻¹). Wird die Probe langsam aufgeheizt, so wandelt sie sich vor Erreichen des Schmelzpunktes in die Mod. I um, die ihrerseits dann bei 148°C schmilzt. Die Umwandlungsenthalpie beträgt 18 J·g⁻¹.

Die DSC- und TGA-Thermogramme wurden unter Verwendung einer DSC 7 und TGA 7 der Fa. Perkin-Elmer erhalten. Die Röntgendiffraktoramme wurden mit einem Stoe Transmissionsdiffraktometer registriert. Die IR-, FIR- und Raman-Spektren wurden mit Fourier-IR Spektrometern IFS 66 (IR), IFS 66v (FIR) und IFS 88 (Raman) der Fa. Bruker aufgenommen. Die ¹³C-Festkörper-NMR-Spektren wurden mit einem Bruker MSL 300 registriert.

Die Kristallmodifikation II des DTTP wird in pharmazeutischen Formulierungen in hoher Reinheit eingesetzt. Aus Stabilitätsgründen sollte Mod. II keine größeren Anteile an Mod. I enthalten. Bevorzugt wird eine Wirkstoffqualität mit weniger als 10 % Mod. I, ganz besonders bevorzugt mit weniger als 5 %.

Die Herstellung der Mod. II erfolgt, indem man DTTP der Modifikation I in Wasser oder inerten organischen Stoffen, z. B. in niederen Alkoholen, Ketonen oder Alkanen bis zum Erreichen des gewünschten Umwandlungsgrades, besonders bevorzugt bis zur quantitativen Umwandlung in die Mod. II suspendiert. In der Regel findet diese Umwandlung bei Temperaturen von 0°C bis 150°C, bevorzugt bei Raumtemperatur bis 100°C, ganz besonders bevorzugt bei 50 - 80°C statt. Die erhaltenen Kristalle der Mod. II werden abgetrennt und zur Entfernung vorhandenen Lösungsmittels bei RT im Vakuum oder erhöhter Temperatur bis zur Gewichtskonstanz getrocknet.

### Beispiel 1

0,6 g DTTP der Mod. I werden in 10 ml n-Heptan suspendiert und bei 80°C zwei Stunden gerührt, der Rückstand abfiltriert und bei 70°C bis zur Gewichtskonstanz getrocknet. Zur Prüfung auf quantitative Umwandlung wird das IR-Spektrum aufgenommen.

### Beispiel 2

0,5 g DTTP der Mod. I wird in 5 ml Methylisobutylketon suspendiert und 2 Stunden bei 60°C gerührt. Der Rückstand wird filtriert und im Trockenschrank bei 120°C bis zur Gewichtskonstanz getrocknet. Die quantitative Umwandlung wird durch Aufnahme des DSC-Thermogramms sichergestellt.

### Beispiel 3

0,5 g DTTP der Mod. I werden mit 5 ml Methanol sowie mit Kristallkeimen der Mod. II versetzt und 2 Stunden unter Rückfluß erwärmt. Der Rückstand wird abfiltriert und bei Raumtemperatur im Vakuum bis zur Massenkonstanz getrocknet. Die quantitative Umwandlung in die stabile Mod. II wird durch Aufnahme eines Röntgendiffraktogramms sichergestellt.

### Beispiel 4

0,5 g DTTP der Mod. I wird in 5 ml Methanol suspendiert und 4 Stunden bei Raumtemperatur gerührt. Der Rückstand wird abfiltriert und bei 60°C bis zur Gewichtskonstanz getrocknet. Die quantitative Umwandlung wird durch Aufnahme eines DSC-Thermogramms sichergestellt.

**Tab. 1:**

| **Leistungskompensationskalorimetrie Differential Scanning Calorimetry** | | |
|---|---|---|
| | Mod. I | Mod. II |
| Schmelzpunkt | 198°C | 178°C |
| Schmelzenthalpie | 82 J g⁻¹ | nicht bestimmt |
| Umwandlungsenthalpie | - | 18 J g⁻¹ |

**Tab. 2:**

| **Röntgendiffraktometrie** | |
|---|---|
| Reflexe | |
| Mod. I [2 Theta] | Mod. II [2 Theta] |
| 8.2 | 9.0 |
| 9.2 | 9.4 |
| 11.4 | 12.7 |
| 13.6 | 13.1 |
| 14.2 | 13.6 |
| 15.9 | 13.9 |
| 17.5 | 16.7 |
| 18.0 | 17.6 |
| 18.5 | 18.0 |
| 18.8 | 18.2 |
| 19.5 | 19.2 |
| 19.8 | 19.5 |
| 21.4 | 20.0 |
| 22.4 | 21.2 |
| 23.3 | 21.6 |
| 23.7 | 23.0 |
| 24.2 | 23.8 |
| 25.0 | 24.2 |
| 25.8 | 25.0 |
| 27.0 | 25.3 |
| 27.9 | 25.6 |
| 29.8 | 26.3 |
| 31.0 | 26.9 |
| 31.7 | 27.4 |
| | 27.9 |
| | 28.4 |
| | 28.9 |
| | 29.5 |
| | 30.4 |
| | 31.8 |
| | 35.7 |
| | 37.6 |
| | 38.9 |

**Tab. 3:**

| **IR-Spektroskopie** | |
|---|---|
| Peakmaxima | |
| Mod. I [cm⁻¹] | Mod. II [cm⁻¹] |
| 3263 | 3259 |
| 3200 | 3188 |
| 3045 | 3148 |
| 1634 | 3042 |
| 1566 | 1631 |
| 1492 | 1558 |
| 1477 | 1489 |
| 1449 | 1477 |
| 1436 | 1449 |
| 1374 | 1371 |
| 1345 | 1352 |
| 1317 | 1315 |
| 1276 | 1274 |
| 1249 | 1249 |
| 1219 | 1214 |
| 1145 | 1143 |
| 1062 | 1120 |
| 859 | 1063 |
| 828 | 859 |
| 818 | 828 |
| 763 | 760 |
| 746 | 745 |
| 728 | 727 |
| 706 | 695 |
| 648 | 649 |

**Tab. 4:**

| ^{**13**}**C-Festkörper-NMR-Spektroskopie** | |
|---|---|
| Peakmaxima | |
| Mod. I [ppm] | Mod. II [ppm] |
| 208.9 | 209.0 |
| 207.0 | 173.1 |
| 200.9 | 157.2 |
| 192.4 | 150.6 |
| 173.6 | 145.9 |
| 156.6 | 134.7 |
| 150.0 | 132.4 |
| 145.1 | 131.0 |
| 135.8 | 129.3 |
| 135.1 | 128.3 |
| 132.4 | 123.3 |
| 131.6 | 122.1 |
| 130.6 | 121.5 |
| 128.8 | 114.5 |
| 122.5 | 108.7 |
| 121.9 | 54.8 |
| 113.9 | 41.4 |
| 107.1 | 39.8 |
| 71.8 | 20.9 |
| 56.7 | 19.2 |
| 53.2 | 17.1 |
| 50.2 | |
| 44.1 | |
| 40.0 | |
| 37.1 | |
| 23.1 | |
| 20.7 | |
| 16.1 | |

**Tab. 5:**

| **FIR-Spektroskopie** | |
|---|---|
| Peakmaxima | |
| Mod. I [cm⁻¹] | Mod. II [cm⁻¹] |
| 482 | 460 |
| 461 | 450 |
| 446 | 442 |
| 439 | 365 |
| 363 | 357 |
| 325 | 331 |
| 294 | 295 |
| 281 | 280 |
| 212 | 220 |
| 120 | 211 |
| 88 | 198 |
| | 117 |

**Tab. 6:**

| **Raman-Spektroskopie** | |
|---|---|
| Peakmaxima | |
| Mod. I [cm⁻¹] | Mod. II [cm⁻¹] |
| 3068 | 3068 |
| 2919 | 2969 |
| 2871 | 2918 |
| 1599 | 2890 |
| 1522 | 2859 |
| 1461 | 1597 |
| 1263 | 1519 |
| 1220 | 1458 |
| 1202 | 1314 |
| 847 | 298 |
| 299 | 168 |
| 198 | 96 |
| 88 | |

## Patentansprüche

1. Thermodynamisch stabile Modifikation von N-[4-[2-(2,4-Dimethylphenoxy)phenyl]-2-thiazolyl]-1,4,5,6-tetrahydro-2-pyrimidinamin (DTTP) mit einem Schmelzpunkt von 178°C (DSC), einer Umwandlungsenthalpie von 18 J/g (DSC, 10 K min⁻¹), Reflexen im Röntgendiffraktogramm bei 19,2; 12,7 und 25,3 (2 Theta), Peakmaxima im IR-Spektrum bei 3188 cm⁻¹ und 1558 cm⁻¹, Peakmaxima im ¹³C-Festkörper-NMR-Spektrum bei 17,1; 157,2 und 123,3 ppm, einem Peakmaximum im FIR-Spektrum bei 220 cm⁻¹, Peakmaxima im Raman-Spektrum bei 96 cm⁻¹, 1314 cm⁻¹ und 2969 cm⁻¹.

2. Verfahren zur Herstellung einer thermodynamisch stabilen Modifikation von DTTP gemäß Anspruch 1, dadurch gekennzeichnet, daß man eine thermodynamisch metastabile Modifikation von DTTP in Wasser oder inerten organischen Stoffen bis zum Erreichen des gewünschten Umwandlungsgrades suspendiert.

3. Arzneimittel enthaltend eine thermodynamisch stabile Modifikation von DTTP gemäß Anspruch 1.

4. Verwendung einer thermodynamisch stabilen Modifikation von DTTP gemäß Anspruch 1 bei der Herstellung von Arzneimitteln.

## Claims

1. Thermodynamically stable modification of N-[4-[2-(2,4-dimethoxyphenoxy)-phenyl]-2-thiazolyl]-1,4,5,6-tetrahydro-2-pyrimidinamine (DTTP), having a melting point of 178°C (DSC), an enthalpy of transformation of 18 J/g (DSC, 10 K min⁻¹), reflections in the X-ray diffraction pattern at 19.2; 12.7 and 25.3 (2 theta), peak maxima in the IR spectrum at 3188 cm⁻¹ and 1558 cm⁻¹, peak maxima in the solid-state ¹³C-NMR spectrum at 17.1; 157.2 and 123.3 ppm, a peak maximum in the FIR spectrum at 220 cm⁻¹, peak maxima in the Raman spectrum at 96 cm⁻¹, 1314 cm⁻¹ and 2969 cm⁻¹.

2. Process for the preparation of a thermodynamically stable modification of DTTP according to Claim 1, characterized in that a thermodynamically metastable modification of DTTP is suspended in water or inert organic substances until the desired conversion is reached.

3. Medicament containing thermodynamically stable modification of DTTP according to Claim 1.

4. Use of a thermodynamically stable modification of DTTP according to Claim 1 in the preparation of medicaments.

## Revendications

1. Forme thermodynamiquement stable de N-[4-[2-(2,4-diméthylphénoxy)phényl]-2-thiazolyl]-1,4,5,6-tétrahydro-2-pyrimidinamine (DTTP) ayant un point de fusion de 178°C (analyse calorimétrique différentielle), une enthalpie de transformation de 18 J/g (analyse calorimétrique différentielle, 10 K min⁻¹), des raies de réflexion dans le diagramme de diffraction de rayons X à 19,2 ; 12,7 et 25,3 (2 thêta), des maximums de pics dans le spectre infrarouge à 3188 cm⁻¹ et 1558 cm⁻¹, des maximums de pics dans le spectre de résonance magnétique des noyaux de ¹³C sur solide à 17,1 ; 157,2 et 123,3 ppm, un maximum de pic dans le spectre de lointain infrarouge à 220 cm⁻¹, des maximums de pics dans le spectre Raman à 96 cm⁻¹, 1314 cm⁻¹ et 2969 cm⁻¹.

2. Procédé de préparation d'une forme thermodynamiquement stable de DTTP suivant la revendication 1, caractérisé en ce qu'on met en suspension une forme thermodynamiquement métastable de DTTP dans l'eau ou dans des substances organiques inertes jusqu'à ce que le degré de transformation souhaité ait été atteint.

3. Médicaments contenant une forme thermodynamiquement stable de DTTP suivant la revendication 1.

4. Utilisation d'une forme thermodynamiquement stable de DTTP suivant la revendication 1 dans la préparation de médicaments.
